# EUROPÄISCHE PATENTSCHRIFT

(11) **EP 1 496 950 B1**
(45) Veröffentlichungstag und Bekanntmachung des Hinweises auf die Patenterteilung: **12.11.2008**
(21) Anmeldenummer: 03706110.8
(22) Anmeldetag: 12.02.2003
(51) Int. Cl.: A61L 9/14, A61L 9/12, B05B 12/12, B05B 12/02

(54) **VERFAHREN ZUM VERSPRÜHEN VON PORTIONEN EINES LUFTVERBESSERNDEN STOFFS**
METHOD FOR SPRAYING PORTIONS OF AN AIR-IMPROVING SUBSTANCE
PROCEDE DE PULVERISATION DE QUANTITES D'UNE MATIERE DESODORISANTE

(30) Priorität: 19.04.2002 AT 6132002
(43) Veröffentlichungstag der Anmeldung: 19.01.2005
(73) Patentinhaber: Hagleitner Hygiene International GmbH, 5700 Zell am See (AT)
(72) Erfinder: HAGLEITNER, Hans, Georg, A-5700 Zell am See (AT)
(74) Vertreter: Torggler, Paul Norbert
(86) Internationale Anmeldenummer: PCT/AT2003/000041
(87) Internationale Veröffentlichungsnummer: WO 2003/089021

(56) Entgegenhaltungen:
- EP-A- 1 494 956
- US-A1- 2002 036 358
- US-B1- 6 254 065
- US-B1- 6 267 297

## Beschreibung

Die Erfindung betrifft Verfahren zum Versprühen von Portionen eines luftverbessernden Stoffs in einem Raum, insbesondere in einem Sanitärraum.

Für die Luftverbesserung insbesondere in Sanitärräumen durch Sprühen eines geruchsbindenden und/oder duftenden Stoffs ist ein programmierbarer Spender (beispielsweise aus US 6,267,297 B) bekannt, bei dem nach verschiedenen, wählbaren Kriterien ablaufende Sprühfolgen vorgegeben werden können. Dieser Spender weist Einstellmöglichkeiten für Beginn und Ende der Sprühfolgen, für die Dauer der zeitlichen Abstände der Sprühvorgänge, für Tag- oder Nachtbetrieb, usw. auf, und sind mit einer elektrischen Sprühknopfbetätigung, einer elektronischen (Zeit-) Steuerung, einer Spannungsversorgung, einem Beleuchtungssensor und einer Einrichtung versehen, die den Aufbrauch des Duftstoffes überwacht. Die Benützeranzahl und die Benützerfrequenz sind dabei nur bedingt berücksichtigt. Sie werden miterfasst, wenn Sanitärräume ohne Tageslicht von einzelnen Personen benützt werden, da das Einschalten der Beleuchtung einen Benützer vermuten lässt.

In öffentlichen Sanitärräumen, insbesondere bei einer hohen Benützerzahl und schwankender Benützerfrequenz sind die bekannten Einrichtungen unbefriedigend. Die Sprühvorgänge sollten immer dann erfolgen, wenn sie notwendig sind, wobei Sprühvorgänge bei Anwesenheit von Benützern vermieden werden sollten, um nicht die Benützer zu besprühen. Eine Zeitsteuerung kann die Anwesenheit eines oder mehrerer Benützer nicht feststellen, und eine Helligkeitssteuerung kann die Anzahl der Benützer nicht ermitteln.

Aus der EP 1 494 956, die am 23. Oktober 2003 als WO 2003/086947 veröffentlicht worden ist, und daher einen Stand der Technik nach Artikel 54 (3) und (4) EPÜ bildet, ist weiters ein Verfahren zum Versprühen von Portionen eines luftverbessernden Stoffs in einem Raum, insbesondere in einem Sanitärraum bekannt geworden, bei dem festgestellt wird, ob sich im Raum Personen bewegen, und wobei ein Sprühvorgang nur ausgelöst wird, wenn keine Bewegung von Personen mehr erkannt wird.

Insbesondere in öffentlichen Sanitärräumen kann aber eine anhaltende, starke Frequenz dazu führen, dass ein für die Auslösung des Sprühvorganges erforderliche Ruhezustand nicht eintritt.

Nach der Erfindung wird nun nicht nur festgestellt, ob sich im Raum Personen bewegen und ein Sprühvorgang ausgelöst, wenn keine Bewegung von Personen mehr erkannt wird, sondern es wird bei einer über einen vorbestimmten Zeitraum anhaltenden Erkennung der Bewegung von Personen im Raum der Sprühvorgang auch dann ausgelöst, wenn eine Bewegung von Personen erkannt wird.

In dieser Betriebsweise ist der Zeitraum vorzugsweise ebenfalls einstellbar, und umfasst beispielsweise 2,5 bis 15 Minuten. Ist in einer reinen Bewegungsmeldesteuerung kein Ruhezustand über einen vorbestimmten Zeitraum von beispielsweise 10 Minuten ermittelbar, was auf eine hohe Benützerzahl schließen lässt, so erfolgt der Sprühvorgang auch mit dem Risiko, dass die gerade anwesenden Benützer besprüht werden. Hält die intensive Benützung über einen längeren Zeitraum, beispielsweise den ganzen Vormittag an, so entsteht eine Sprühfolge mit zeitlich gleichen Abständen, die jedoch, aufgrund der hohen Benützerfrequenz auch notwendig, wesentlich kürzer sind als bei einer Intervallsteuerung, und nur so lange beibehalten wird, als diese Benützerfrequenz gegeben ist.

Eine Einrichtung zum Versprühen von Portionen eines luftverbessernden Stoffs, beispielsweise eines geruchsbindenden Stoffs, eines Duftstoffs od. dgl., die beide Betriebsweisen ermöglicht, ist mit einem Bewegungssensor versehen. Damit eine erkannte Person den Raum verlassen kann, sodass sie nicht besprüht wird, ist zwischen dem Erkennen einer Bewegung und einer Signalabgabe zur Auslösung eines Sprühvorganges eine Verzögerung vorgesehen. Die vorzugsweise einstellbare Verzögerungszeit liegt zwischen 15 und 30 Sekunden. Die Verzögerung kann, je nach Ausführung, am Bewegungssensor eingestellt werden, sodass die Bewegungsmeldung an die Steuerung verzögert erfolgt oder von der Steuerung selbst eingestellt werden. In beiden Fällen wird die Anwesenheit einer sich bewegenden Person erneut vom Bewegungssensor erkannt, und die nächste Verzögerungszeit beginnt zu laufen, usw. Somit wird das Auslösesignal für den Sprühvorgang immer erst nach, gegebenenfalls sogar erst nach oftmaliger Bewegungserkennung und nur dann abgegeben, wenn die Bewegung bereits beendet ist. Wenn die Verzögerung am Bewegungssensor erfolgt, so stellt dessen Steuerungssignal eine Bewegungsendemeldung dar.

Nachstehend wird nun die Erfindung anhand der Figuren der beiliegenden Zeichnung näher beschrieben, ohne darauf beschränkt zu sein.

Es zeigen:
- Fig. 1: eine schematische Vorderansicht einer Einrichtung zum Versprühen von Portionen eines luftverbessernden Stoffs, und
- Fig. 2 bis 4: Funktionsdiagramme von drei verschiedenen Betriebsweisen.

Eine Einrichtung zum Versprühen eines luftverbessernden Stoffs in einem Raum, insbesondere in einem Sanitärraum, weist ein an einer Wand od. dgl. fixierbares Gehäuse 1 auf, in dem ein mit einem Sprühkopf 3 versehener, auswechselbarer Behälter 2 angeordnet ist, der den luftverbessernden Stoff enthält. Der Behälterinhalt wird mittels eines Hilfsmittels in jedem Sprühvorgang durch ein Fenster 4 des Gehäuses 1 ausgesprüht, beispielsweise mittels einer elektrisch angetriebenen Pumpvorrichtung. Als Stromquelle sind im Gehäuse 1 nicht gezeigte Batterien od. dgl. enthalten; es ist aber auch ein Anschluss an das Stromnetz möglich.

Das Gehäuse 1 enthält weiters eine elektronische Steuerung 5, die einen Betriebsweisenwählschalter 6, Leuchtdioden 7 für Zustands-, Füllstands- und Betriebsanzeige, Zeiteinstelldrehknöpfe 8 für die Einstellung der Intervalle in der zweiten und in der dritten Betriebsweise, einen Bewegungssensor 9 mit einer Verzögerungszeit Δt für die Signalabgabe, die insbesondere eingestellt werden kann, und einen lichtempfindlichen Sensor 11 umfasst, über den beispielsweise ein Tag-/Nachtbetrieb festgelegt werden kann. Schließlich ist noch ein Testmodulschalter 10 vorgesehen, wobei in einer ersten Schalterstellung die Grundfunktionen der Einrichtung und in einer zweiten Schalterstellung die Grundeinstellungen der Steuerung 5, des Bewegungssensors 9, des lichtempfindlichen Sensors 11, usw. geprüft werden können.

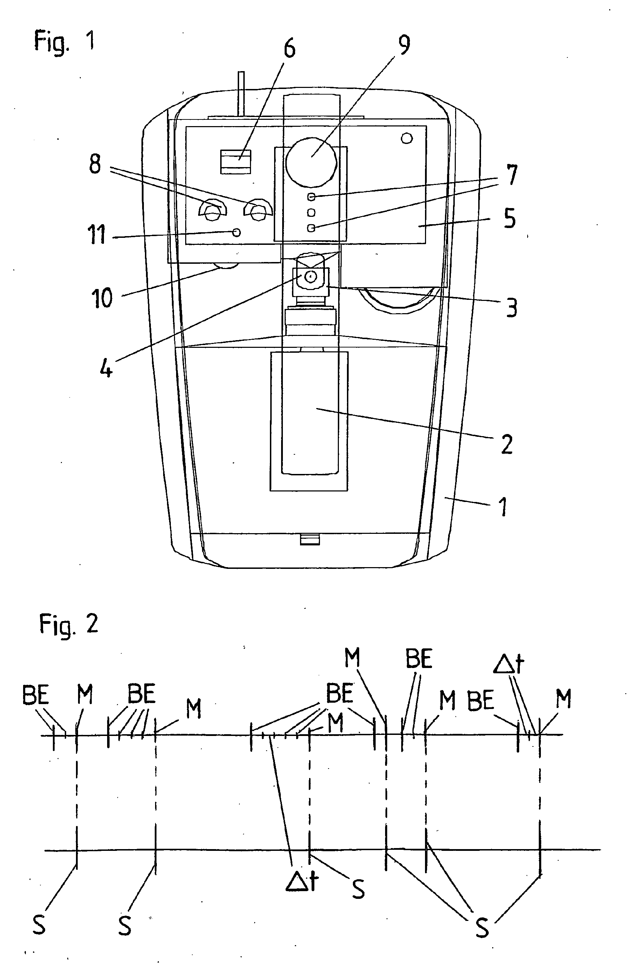

Die Einrichtung zum Versprühen eines luftverbessernden Stoffes kann dank des Bewegungssensors 9 dem Bedarf angepasst auf unterschiedliche Arten betrieben werden. Ein Funktionsdiagramm der ersten Betriebsart ist in Fig. 2 gezeigt. Jeder Sprühvorgang S wird ausschließlich als Folge einer erkannten Bewegung BE eines Benützers oder einer sonstigen Person ausgelöst, sobald diese Bewegung nicht mehr festgestellt wird. Hierzu kann am Bewegungssensor 9 eine Verzögerungszeit Δt eingestellt werden. Erkennt der im Anschluss an die Verzögerungszeit Δt wieder aktiv geschaltete Bewegungssensor 9 wiederum eine Bewegung BE, so beginnt die nächste Verzögerungszeit At zu laufen, nach der entweder das Meldesignal M übermittelt oder ein weiteres Mal eine Bewegung BE erkannt wird. Die Verzögerungszeit At ist so gewählt, dass der Benützer den Raum verlassen kann, und er keinesfalls mit dem luftverbessernden Stoff besprüht wird.

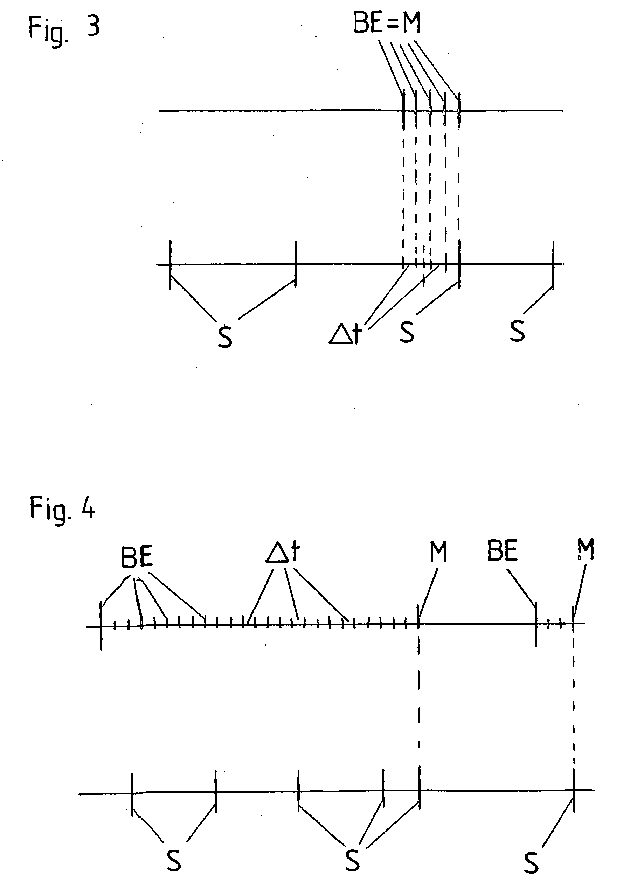

Fig. 3 zeigt eine zweite Betriebsweise, in der ein Zeitintervall vorgegeben ist, und Sprühvorgänge S in größeren Abständen automatisch erfolgen, soferne der Bewegungssensor 9 kein Signal abgibt. Das bedeutet, dass ein Sprühvorgang unterdrückt wird, wenn gerade zum vorgegebenen Sprühzeitpunkt vom Bewegungssensor P eine Bewegung BE erkannt wird. Bevorzugt wird der unterdrückte Sprühvorgang S nach dem Ende der Bewegung nachgeholt.

Fig. 4 zeigt ein Funktionsdiagramm einer dritten Betriebsweise, die für eine sehr hohe Benützerfrequenz ausgelegt ist. Hier wird es aufgrund einer sich ständig wiederholenden Bewegungserkennung BE über längere Zeit zu keinem Meldesignal M für das Ende der Bewegung und zu keinem Sprühvorgang kommen. Für diesen Fall wird die Bewegungsmeldesteuerung aus Fig. 2 übersteuert, indem nach einer mittels des Drehknopfes 8 einstellbaren Fixzeit ein Sprühvorgang S erfolgt, obwohl Personen im Raum sind.

## Patentansprüche

1. Verfahren zum Versprühen von Portionen eines luftverbessernden Stoffs in einem Raum, insbesondere in einem Sanitärraum, **dadurch gekennzeichnet, dass** festgestellt wird, ob sich im Raum Personen bewegen, und ein Sprühvorgang erst ausgelöst wird, wenn keine Bewegung von Personen mehr erkannt wird, wobei bei einer über einen vorbestimmten Zeitraum anhaltenden Erkennung der Bewegung von Personen im Raum der Sprühvorgang auch dann ausgelöst wird, wenn eine Bewegung von Personen erkannt wird.

## Claims

1. A method of spraying portions of an air-improving substance in a room, in particular in a washroom, **characterised in that** it is detected that persons are moving in the room and that the next spray operation is triggered off only when no more movement of persons is detected, wherein in the case of a movement of persons in the room, which continues over a predetermined period of time, the spray operation is triggered even when a movement of persons is detected.

## Revendications

1. Procédé de pulvérisation de quantité d'une matière désodorisante dans un espace. En particulier dans un local sanitaire, **caractérisé en ce que** l'on constate si des personnes se dèplacent dans l'espace, et, ensuite, on déclenche un processus de pulverisation si plus aucun movement de personnes n'est constaté, dans lequel, en cas d'identification de movement de personnes dans l'espace persistant durant une période de temps predetermine, le processus de pulverisation est également déclenché ensuite, lorsqu'un movement de personnes est identifié.
